# EUROPÄISCHE PATENTSCHRIFT

(11) **EP 0 948 961 B1**
(45) Veröffentlichungstag und Bekanntmachung des Hinweises auf die Patenterteilung: **16.06.2004**
(21) Anmeldenummer: 99106452.8
(22) Anmeldetag: 29.03.1999
(51) Int. Cl.: A61K 9/46, A61K 9/20, A61K 31/495, A61K 45/00

(54) **Calciumhaltige Brausetablette mit einem Antihistaminikum als Wirkstoff**
Effervescent tablet comprising Calcium with an antihistaminic as active agent
Comprimé effervescent comprenant du calcium avec un antihistaminique comme principe actif

(30) Priorität: 31.03.1998 DE 19814392
(43) Veröffentlichungstag der Anmeldung: 13.10.1999
(73) Patentinhaber: HERMES Fabrik pharmazeutischer Präparate Franz Gradinger GmbH & Co., 82049 Grosshesselohe/München (DE)
(72) Erfinder: Rothenberger, Siegfried Dr., 81475 München (DE); Dandl, Klaus Dr., 82041 Deisenhofen (DE); Hein, Thomas Dr., 83623 Dietramzell (DE)
(74) Vertreter: VOSSIUS & PARTNER

(56) Entgegenhaltungen:
- EP-A- 0 418 564
- CA-A- 2 084 028
- DOOTZ H ET AL: "Rote Liste 1997" 1997 , ECV EDITIO CANTOR VERLAG FÜR MEDIZIN UND NATURWISSENSCHAFTEN GMBH , AULENDORF XP002159456 07015
- DOOTZ H ET AL: "Rote Liste 1997" 1997 , ECV EDITION CANTOR VERLAG FÜR MEDIZIN UND NATURWISSENSCHAFTEN GMBH , AULENDORF XP002159457 62020

## Beschreibung

Gegenstand der vorliegenden Erfindung ist eine calciumhaltige Brausetablette, die das Antihistaminikum Cetirizin als Wirkstoff umfaßt.

Bedingt durch das in den letzten Jahren verstärkte Auftreten von allergischen Reaktionen auch besonders bei Kindern kommt der Behandlung mit Antihistaminika zunehmende Bedeutung zu. Diesbezüglich sind unterschiedliche Applikationsformen entwickelt worden. Zu nennen sind in diesem Zusammenhang insbesondere Antihistaminika enthaltende Kapseln und Tabletten, die den Wirkstoff über Stunden dosiert freisetzen, sobald sie im Körper zersetzt werden.

Nachteilig an dieser Applikationsform ist insbesondere der z.T. bittere Geschmack der Tabletten, sowie Erschwernisse beim Schlucken.

Bei akuten allergischen Reaktionen hat sich die Applikation von Antihistaminika über Spritzen bewährt. Diese sind aber regelmäßig durch einen Arzt zu verabreichen, der gerade in dringenden Fällen oft nicht unmittelbar erreicht werden kann. Zudem ist diese Applikationsform mit nicht unerheblichen Schmerzen verbunden, wenn die Spritzennadel nicht fachkundig gesetzt wird.

EP 0 418 564 offenbart eine Brausetablette zur Anwendung bei Erkältung oder Allergien mit einem reduzierten Natriumgehalt.

CA 2,084,028 offenbart unter anderem eine Kombination von Calcium und Acetaminophen.

Calciumbrausetabletten und Brausetabletten welche das Antihistaminikum Loratidin enthalten sind unter anderem unter der Bezeichnung Calcium Sandoz ® bzw. Lisino ® in Deutschland auf dem Markt.

Folglich besteht ein Bedürfnis nach einer neuen Applikationsform für Cetirizin, die bei den Betroffenen eine hohe Akzeptanz erwarten läßt und desweiteren auch in akuten Fällen zuverlässig wirkt, eine gute Bioverfügbarkeit aufweist und den Wirkstoff schnell verfügbar macht.

Diese Aufgabe wird erfindungsgemäß durch eine calciumhaltige Brausetablette gelöst, die als Wirkstoff Cetirizin enthält.

Vorteilhaft an dieser oralen Applikationsform ist insbesondere die Möglichkeit, die organoleptischen Eigenschaften in weitem Umfang einstellen zu können.

Desweiteren wurde gefunden, daß die antiallergische Wirksamkeit von Cetirizin deutlich durch die Verabreichung in Verbindung mit Calcium gesteigert werden kann.

Dieses Phänomen ist vermutlich darauf zurückzuführen, daß Calciumionen eine stabilisierende Wirkung auf die Mastzellen entfalten können, was zu einer Verminderung der Antikörpervermittelten Degranulationsreaktion und damit zur Verminderung der Histaminfreisetzung führt.

Überraschenderweise wurde ferner gefunden, daß die Bioverfügbarkeit von Cetirizin durch die gleichzeitige Verabreichung mit calciumhaltigen Verbindungen nicht oder nur unwesentlich beeinträchtigt wird, obwohl die Bildung von nicht oder nur schlecht resorbierbaren Komplexen aus Calciumionen und Cetirizin nicht auszuschließen ist.

Schließlich ist hervorzuheben, daß die Resorption des Wirkstoffs bedingt durch dessen Vorliegen in Lösung rasch erfolgen kann. Zudem läßt die Verabreichung des Wirkstoffs in einer Flüssigkeit oder in Form von Kau- oder Lutschtabletten eine hohe Akzeptanz bei den betroffenen Verkehrskreisen erwarten.

Unter dem Begriff "Brausetablette" ist in der vorliegenden Erfindung jegliche Formulierung zu verstehen, die bei Kontakt mit einer geeigneten Flüssigkeit ein Gas, vorzugsweise Kohlendioxid, freisetzt. Umfaßt werden somit von dieser Definition klassische Tabletten jeglicher Form und Gestalt, Pulver und Granulate, die vorzugsweise ein Brausemittel in Kombination mit einer Säure enthalten.

Als CO₂-abspaltende Substanzen können die üblichen CO₂-Entwickler eingesetzt werden, vorzugsweise Hydrogencarbonat, Carbonat von Alkali- oder Erdalkalielementen oder Gemische davon, besonders bevorzugt in Form der Natrium, Kalium, Magnesium und/oder Calciumsalze.

Als Säuren eignen sich alle eßbaren Säuren, die üblicherweise in Brausezubereitungen zur Erzeugung von CO₂ als einem Brausemittel eingesetzt werden. Bevorzugt verwendet werden Acidum tartaricum, Acidum citricum, Adipinsäure, Fumarsäure, Maleinsäure, gegebenenfalls auch deren Anhydride, soweit herstellbar.

Neben den Brausemitteln und Säuren können die erfindungsgemäßen Brausetabletten weitere übliche Zusätze wie Farbstoffe, Geschmackskorrigentia, Zerfallshilfen, Schmiermittel, Füllstoffe, Hydrokolloide (Maltodextrin oder Dextrine) Antioxidantien, grenzflächenaktiven Substanzen und/oder Bindemittel enthalten.

Als Geschmackskorrigentia kommen alle üblichen Aromastoffe, wie Limonen-, Grapefruit-, Orangen- oder Sauerkirscharoma sowie Süßungsmittel in Betracht, wie Saccharin, Cyclamat, Aspartam, Acesulfam K, Sorbit, Mannit, Mannitol, Sorbitol oder Gemische davon.

Als geeignete Flüssigkeiten sind z.B. Wasser, Fruchtsäfte oder Tees zu nennen. Auch Speichelflüssigkeit ist denkbar.

Das Verhältnis von CO₂-abspaltenden Substanzen zu Säure liegt üblicherweise im Bereich von 1 Teil zu 1-3 Teilen, vorzugsweise im Bereich von 1 zu 1,5-2,5.

Der Calciumgehalt pro Tablette bzw. Darreichungsform beträgt ca. 7 bis 30 Gew.-%, insbesondere ca. 10 bis 25 Gew.-% und liegt üblicherweise zwischen 400 und 1500 mg Calcium, bevorzugt zwischen 400 und 1200 mg.

Weitere übliche Zusatzstoffe und Mischungsverhältnisse sind in US-A-5,503,846 aufgeführt.

Calcium liegt in der erfindungsgemäßen Brausetablette in Form eines biologisch verträglichen Salzes vor, wobei vorzugsweise Calciumcarbonat, Calciumhydrogencarbonat, Calciumgluconat, Calciumsaccharat, Calciumcitrat, Calciumphosphat, Ca-Salze der Heptagluconsäure, Lactobionsäure, Lävulinsäure, Milchsäure und/oder Ansparaginsäure, sowie Gemische davon verwendet werden.

Üblicherweise enthält eine erfindungsgemäße Brausetablette zwischen 5 und 10 mg Cetirizin-2HCl.

Die erfindungsgemäßen calciumhaltigen Brausetabletten stellen eine neue, verbesserte Applikationsform für Cetirizin dar, die eine gute Bioverträglichkeit gewährleistet, verbesserte organoleptische Eigenschaften besitzt, ohne Problem selbst von Kindern angewendet werden kann, eine rasche Aufnahme des Wirkstoffs ermöglicht, und nicht zuletzt durch die gleichzeitige Verabreichung von Calcium zu einer gesteigerten Wirksamkeit von Cetirizin führt.

Die nachstehenden Beispiele erläutern die Erfindung.

### Beispiel 1:

2250 Teile Citronensäure, 150 Teile Natriumhydrogencarbonat, 100 Teile Äpfelsäure, Natiumcyclamat, Saccharin-Natrium und 1250 Teile Calciumcarbonat werden mit Wasser so granuliert, daß die hauptsächlich kristallierten Citronensäurepartikel sowie die Äpfelsäure und die Süßstoffe in einer Schicht aus dem durch die Reaktion entstandenen Calciumcitrat und unreagiertem Calciumcarbonat umhüllt sind. Anschließend wird das Brausegranulat bei 70 °C oder mittels Vakuum bei 60 °C und 15 mbar getrocknet.

10 Teile Cetirizin-2HCl werden mit 30 Teilen Maltodextrin und Aroma intensiv gemischt und anschließend in mehreren Schritten zum Brausegranulat gemischt.

Aus der resultierenden Endmischung werden Tabletten mit einem Durchmesser von 25 mm und einem Gewischt von ca. 4g gepreßt.

### Beispiel 2

Es können als Granulierflüssigkeit auch Ethanol oder Ethanol/Wasser-Mischungen verwendet werden; das Granulat wird dann entsprechend Beispiel 1 hergestellt.

### Beispiel 3

2300 Teile Citronensäure, Natiumcyclamat, Saccharin-Natrium und 1250 Teile Calciumcarbonat werden mit Wasser so granuliert, daß die hauptsächlich kristallierten Citronensäurepartikel sowie die Äpfelsäure und die Süßstoffe in einer Schicht aus dem durch die Reaktion entstandenen Calciumcitrat und unreagiertem Calciumcarbonat umhüllt sind. Anschließend wird das Brausegranulat bei 70 °C oder mittels Vakuum bei 60 °C und 15 mbar getrocknet.

10 Teile Cetirizin-2HCl werden mit 30 Teilen Maltodextrin und Aroma intensiv gemischt und anschließend in mehreren Schritten zum Brausegranulat gemischt.

Aus der resultierenden Endmischung werden Tabletten mit einem Durchmesser von 25 mm und einem Gewischt von ca. 4g gepreßt.

Besonders wichtig für die Löslichkeit des Wirkstoffes Cetirizin-2HCl ist die Einhaltung eines niedrigen pH-Wertes der in Wasser aufgelösten Brausetablette, da es im neutralen oder alkalischen Bereich zu einer Ausfällung der Wirkstoffbase kommen kann. Besonders empfehlenswert für eine stabile Lösung sind pH-Werte unter 4,5. Dies wird durch die spezielle Auswahl der Zusammensetzung gemäß Beispiel 1 oder 2 erreicht. Bei den pH-Verhältnissen in Verbindung mit einer sorgfältig ausgewählten Aromatisierung und Süssung der Brausetabletten läßt sich der bittere Geschmack des Wirkstoffs so kaschieren, daß eine für den Verbraucher akzeptable Lösung nach Auflösen in kaltem Wasser entsteht.

Der in den Beispielen 1 und 2 genannte Granulierungsschritt ist nicht in jedem Falle erforderlich. Durch Zusatz geeigneter Mengen an Bindemitteln wie beispielsweise Sorbitol, Mannitol oder Xylitol kann durch Vermischung mit den üblichen, für Brausetabletten einsetzbaren Hilfstoffe das resultierende Gemisch auch direkt verpreßt werden.

## Patentansprüche

1. Calciumhaltige Brausetablette mit Cetirizin, wobei der Calciumgehalt pro Tablette zwischen 400 und 1200 mg Calcium liegt.

2. Calciumhaltige Brausetablette mit Cetirizin als Wirkstoff und einer Brausekomponente, bestehend aus Säuren und Erdalkali- oder Alkalihydrogencarbonaten und/oder Erdalkali- oder Alkalicarbonaten.

3. Calciumhaltige Brausetablette nach einem der vorstehenden Ansprüche, wobei das Calcium in Form von Calciumcarbonat und/oder Calciumcitrat vorliegt.

4. Calciumhaltige Brausetablette nach einem der vorstehenden Ansprüche, wobei die Brausetablette ferner einen oder mehrere Bestandteile enthält, ausgewählt aus Farbstoffen, Geschmackskorrigenzien, Zerfallshilfen, Füllstoffen, Bindemitteln, Antioxidantien, grenzflächenaktiven Substanzen und Schmiermitteln.

5. Calciumhaltige Brausetablette nach einem der vorstehenden Ansprüche, wobei der Gehalt an Cetirizin-2HCl zwischen 5 und 10 mg pro Tablette liegt.

## Claims

1. Calcium-containing effervescent tablet with cetirizine wherein the calcium content per tablet is between 400 and 1,200 mg calcium.

2. Calcium-containing effervescent tablet with cetirizine as active agent and an effervescent component consisting of acids and alkaline-earth or alkaline hydrogencarbonates and/or alkaline-earth or alkaline carbonates.

3. Calcium-containing effervescent tablet according to any preceding claim wherein the calcium is present in the form of calcium carbonate and/or calcium citrate.

4. Calcium-containing effervescent tablet according to any preceding claim wherein the effervescent tablet furthermore contains one or more constituents selected from colouring agents, taste correctors, disintegration aids, fillers, binding agents, antioxidants, surfactants and lubricants.

5. Calcium-containing effervescent tablet according to any preceding claim wherein the content of cetirizine 2HCl is between 5 and 10 mg per tablet.

## Revendications

1. Comprimé effervescent de Cétirizine contenant du calcium, dans lequel la teneur en calcium par comprimé est comprise entre 400 et 1200 mg de calcium.

2. Comprimé effervescent de Cétirizine en tant qu'agent actif contenant du calcium et un composant effervescent constitué d'acides et d'hydrogénocarbonates de métaux alcalino-terreux ou alcalins et/ou de carbonates de métaux alcalino-terreux ou alcalins.

3. Comprimé effervescent contenant du calcium selon l'une quelconque des revendications précédentes, dans lequel le calcium est sous forme de carbonate de calcium et/ou de citrate de calcium.

4. Comprimé effervescent contenant du calcium selon l'une quelconque des revendications précédentes, dans lequel le comprimé effervescent comprend en outre un ou plusieurs constituants choisis parmi les colorants, correcteurs de goût, agents délitants, charges, liants, anti-oxydants, substances tensio-actives et lubrifiants.

5. Comprimé effervescent contenant du calcium selon l'une quelconque des revendications précédentes, dans lequel la teneur en Cétirizine 2HCl est comprise entre 5 et 10 mg par comprimé.
